# EUROPEAN PATENT APPLICATION

(11) **EP 4 764 480 A1**
(43) Date of publication of application: **24.06.2026**
(21) Application number: 24220872.6
(22) Date of filing: 18.12.2024
(51) Int. Cl.: G01N 27/12, G01N 33/00, H01M 8/0662

(54) **SENSOR UNIT FOR AIR FILTER AND METHOD OF MANUFACTURING SENSOR UNIT**

(71) Applicant: MANN+HUMMEL GmbH, 71636 Ludwigsburg (DE); fem Forschungsinstitut, 73525 Schwäbisch Gmünd (DE); Institut für Umwelt & Energie, Technik & Analytik e.V., 47229 Duisburg (DE)
(72) Inventor: HARENBROCK, Dr. Michael, 71636 Ludwigsburg (DE); SRADNICK, Holger, 71636 Ludwigsburg (DE); HEILMANN, Tanja, 71636 Ludwigsburg (DE); BANKODAD, Ahmed, 47229 Duisburg (DE); HAEP, Stefan, 47229 Duisburg (DE); OPIOLKA, Siegfried, 47229 Duisburg (DE); EGETENMEYER, Ann-Kathrin, 73525 Schwäbisch Gmünd (DE); LANZINGER, Gloria, 73525 Schwäbisch Gmünd (DE); FREISINGER, Birger, 73525 Schwäbisch Gmünd (DE)
(74) Representative: Mann + Hummel Intellectual Property

(57) **Abstract**

A sensor unit (20) for an air filter (1) configured to separate, from an air flow (3), at least one harmful gas, in particular for a fuel cell (2), the sensor unit (20) comprising a first sensor (21) and a second sensor (22), each of which comprising two electrodes (27, 28) and a material layer (30) connecting the two electrodes (27, 28), an electrical resistance of the material layer (30) changing when the material layer (30) comes into contact with the harmful gas, and the material layer (30) of the first sensor (21) being different from the material layer (30) of the second sensor (22), and a processing unit (23) configured to determine a time derivative of a change in the electrical resistance of the material layer (30) of at least the first sensor (21), and to generate an output signal (26) based on the determined time derivative.

## Description

### Technical Field

Embodiments to a sensor unit for an air filter configured to separate at least one harmful gas from an air flow. The embodiments also relate to a method of manufacturing the sensor unit.

### Background Art

The working life of a fuel cell depends to a large extent on the cleanliness of the air, which is necessary for the chemical reaction that takes place in the fuel cell to generate electrical energy. Air filters are therefore used to separate harmful gases such as NH3, NO2, and SO2, which reduce the service life of the fuel cell, from the air flow for the fuel cell. Air filters with filter elements containing activated carbon to adsorb the pollutants are used for this purpose.

When the activated carbon has reached full saturation, adsorption of the harmful gases is no longer possible. The harmful gases then enter the fuel cell unfiltered. This filter breakthrough should be avoided or decreased to prevent damage to the fuel cell. The sensor unit is used to detect the filter breakthrough. If the sensor unit detects a filter breakthrough, the saturated filter element can be replaced to not exceed maximum tolerable concentrations of the harmful gases downstream. This enables predictive maintenance of the air filter.

The use of conductive polymers such as polyaniline for a sensor of the sensor unit is known, for example, from US 2008/0101994 A1. The sensor has two electrodes and a material layer of polyaniline connecting the electrodes. The electrical resistance of the material layer changes when the material layer comes into contact with the harmful gas. In order to be able to distinguish between the individual harmful gases, it is also known from the prior art that the sensor unit has a first sensor and a second sensor. The material layers of the two sensors are designed differently so that the sensors show different and gas-specific reactions.

The sensor unit should be able to detect the pollutants preferably in the trace concentration range (ppm and preferably ppb range). In addition, the influence of temperature and humidity, the mathematical compensation of which is known from the prior art (see EP 1 726 964 B1), should be easily controllable in the reliable and preferably selective detection of the harmful gases. In addition, the provision and operation of the sensor unit should be as simple and cost-effective as possible.

### Summary

The embodiments are therefore based on the object to provide a sensor unit for an air filter, which can be manufactured cost-effectively and which enables reliable detection of harmful gases, in particular NH3, NO2, and/or SO2.

The object underlying the embodiments is solved by the combination of features according to claim 1. The embodiments can be taken from the subclaims to claim 1.

According to the embodiments, the sensor unit for an air filter configured to separate, from an air flow, at least one harmful gas, in particular for a fuel cell, comprises a first sensor and a second sensor, each of which comprising two electrodes and a material layer of polyaniline connecting the two electrodes, an electrical resistance of the material layer changing when the material layer comes into contact with the harmful gas, and the material layer of the first sensor being different from the material layer of the second sensor, and a processing unit configured to determine a time derivative of a change in the electrical resistance of the material layer of at least the first sensor. Preferably, the processing unit is configured to determine the time derivative of the change in resistance of the material layer of the second sensor as well. The processing unit then generates an output signal based on the determined time derivative of the change in the electrical resistance of the first sensor and preferably also of the second sensor. The output signal can be used to send a message to the operator of the air filter or to the operator of the air filter/fuel cell combination, for example, that the amount of harmful gases detected is too high and that the filter element needs to be replaced. The output signal can take a wide variety of forms. It can be acoustic, visual or in the form of a data packet, which is then transmitted to a device for further processing via a suitable interface.

In one embodiment, the time derivative or the speed of the relative change in resistance at the first sensor and/or second sensor is used. When determining the relative change in resistance, the measured resistance R is related to an initial resistance R0 of the material layer. The initial resistance R0 can be the resistance that the material layer made of polyaniline has before initial exposure to the harmful gas. If the resistance R increases by 20% compared to the initial resistance R0 as a result of exposure to the harmful gas, for example, the relative change in resistance R/RO assumes the value 1.2.

In the case of the harmful gas NH3, it has been found that with a time-limited exposure to the harmful gas (for example with an exposure duration of one minute or several minutes), the value R/RO rises directly at the beginning of the exposure and falls again after the end of the exposure, but does not return to the initial value (R/RO = 1) even after a longer period of time after the exposure. Due to this drift of the sensor signal, which adds up if the time-limited exposure is repeated several times with corresponding pauses, a simple correlation between the amount of harmful gas during the exposure and the sensor response based on the relative change in resistance is not possible. If, on the other hand, the time derivative of the relative change in resistance is used, the drift of the sensor signal does not affect the measurement results. By using the time derivative of the relative change in resistance Δ(R/R0)/Δt, the influence of the sensor signal drift observed with NH3 can be eliminated and the measurement accuracy can be improved.

If the concentration of NH3 is continuously increased from exposure to exposure, a good approximation of a linear correlation between the harmful gas quantity/harmful gas concentration and the time derivative is obtained in one embodiment. An increase in R/RO can be recognized when the exposure duration is increased. This leads to problems if a correlation between R/RO and the harmful gas concentration is to be established. The time derivative Δ(R/R0)/Δt, on the other hand, is independent of the exposure duration.

Another advantage of using the time derivative of the change in resistance is that mathematical compensation of the influence of relative humidity and temperature is not necessary. In practice, the time derivative of the relative change in resistance is independent of the influence of relative humidity and temperature, as both environmental parameters only change comparatively slowly in practice. The use of the time derivative of the change in resistance increases the measurement accuracy and simplifies the handling of the sensor unit.

In the case of the harmful gas NO2, it has been shown that the resistance of the polyaniline material layer also increases as a result of a temporary exposure to the harmful gas, but that the resistance remains practically constant after the end of the exposure, even after a longer waiting period, and does not drop back to R0. When re-exposed to the same concentration of harmful gas, R/RO increases again by the same order of magnitude, so that a cascaded increase in R/RO can be recognized. This makes it difficult to find a suitable correlation between R/RO and the harmful gas concentration. However, if the time derivative Δ(R/R0)/Δt is used, it is easier to draw conclusions about the harmful gas concentration.

In one embodiment example, the processing unit may be configured to generate the output signal based on the change in the electrical resistance of the material layer of the first sensor exceeding a threshold value S1. Only if, for example, the relative change in resistance R/RO is greater than S1 (and other conditions may be fulfilled) does the processing unit of the sensor unit generate the output signal.

The processing unit can be configured to generate the output signal based on the time derivative of the change in the electrical resistance of the first sensor exceeding a threshold value S2. In one embodiment example, the output signal depends only on this condition. However, the generation of the output signal can also depend on further conditions, preferably on the condition that the change in resistance is greater than S1.

The processing unit can be configured to generate the output signal based on the time derivative of the change in the electrical resistance of the material layer of the first sensor (and preferably also of the second sensor) falling below a negative threshold value S3. This criterion can be used to differentiate between NH3 and NO2. While the resistance of the material layer of a sensor decreases again after exposure to NH3 has ended and the time derivative of the relative change in resistance therefore assumes values less than zero, the resistance remains at an increased level during exposure to NO2 and does not decrease even when the exposure has ended and NO2 is no longer present on the material layer. Accordingly, the time derivative here shows practically no negative values or only negative values that are close to zero, but whose absolute value is not greater than the absolute value of the negative threshold value S3. These principles can be also applied to detecting SO2 with a third sensor.

A further object of the embodiments, the provision of a method of manufacturing the sensor unit described above, is solved by the combination of features according to claim 5. Embodiments can be taken from the subclaims to claim 5.

The method comprises subjecting material layer of the first sensor to a surface treatment with an acid. Insofar as the two sensors and their material layer have been manufactured from polyaniline in an identical manner, this surface treatment constitutes the difference between the two material layers. In one embodiment, the surface treatment is only carried out on the material layer of the first sensor, while no surface treatment is provided for the material layer of the second sensor. However, it is also conceivable that the material layer of the second sensor also undergoes a surface treatment that is different from the surface treatment of the material layer of the first sensor. Here too, the two material layers can differ and can provide different sensor signals when they are exposed to the same harmful gas.

For example, the acid may comprise one among sulphuric acid (H2SO4), hydrochloric acid (HCl), nitric acid (HNO3), phosphoric acid (H3PO4), and sulphonic acid. The surface treatment can include immersing the material layer of the first sensor in an aqueous solution of the acid. This can be a 1-molar to 10-molar solution of the acid (1 mol/l to 10 mol/l or preferably 3 mol/l to 7 mol/l).

The material layer can be immersed in the acid or the aqueous solution of the acid for 5 to 90 seconds for the surface treatment, preferably for 10 to 60 or 20 to 40 seconds. Immersion is preferably carried out without applying a potential.

The method may further comprise building up the material layer by applying a dynamic potential to aniline in an aqueous solution of the acid so that the aniline is polymerised. Preferably, polyaniline is formed in individual, superimposed films on a plate-shaped carrier on which the electrodes are arranged. In one embodiment, the electrodes are interdigital electrodes.

The method may further comprise functionalizing the material layer by adding one among metals, metal alloys, and metal oxides (Pd, Ag, Pd/Sn, Zn) into the aqueous solution. For example, it is possible for the material layer of the first sensor to have at least one functionalized film, which the material layer of the second sensor does not have.

The method may further comprise performing chemical metal deposition on the material layer by immersing the material layer in a dispersion comprising a solvent and metal nanoparticles.

### Brief Description of Drawings

The embodiments are explained in more detail with reference to the drawings.
Figure 1 shows schematically an air filter and a fuel cell.
Figure 2 shows the air filter with a first sensor and a second sensor.
Figure 3 shows the first sensor with two electrodes and a material layer of polyaniline.
Figure 4 shows the variation of exposure to the harmful gases NH3 and NO2.
Figure 5 shows the variation of the relative change in resistance over time.
Figure 6 shows the variation of the time derivative of the relative change in resistance.

### Description of Embodiments

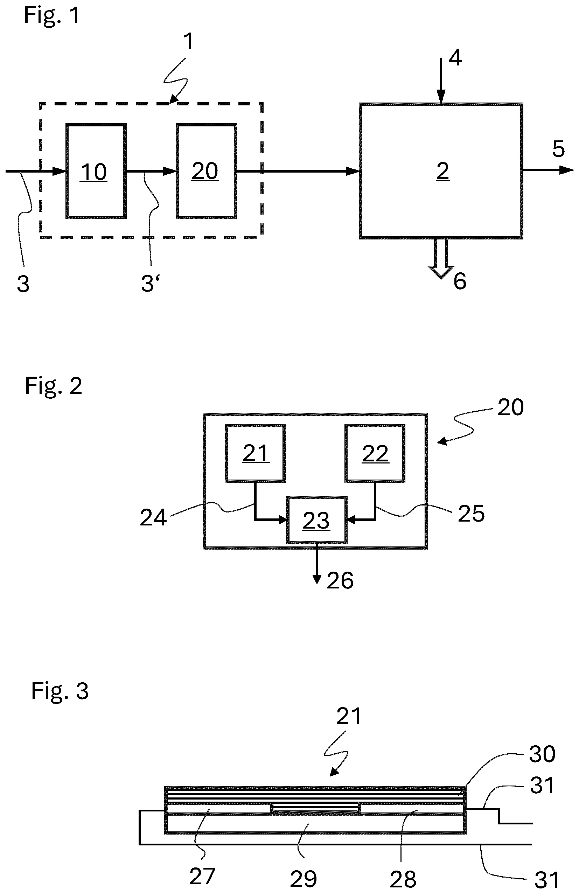

Figure 1 shows an air filter 1 and a downstream fuel cell 2. The air filter 1 comprises a filter element 10 and a sensor unit 20. An air flow 3 enters the air filter 1 and passes through the filter element 10. Harmful gases such as nitrogen dioxide (NO2), ammonia (NH3), and/or sulfur dioxide (SO2) are separated from the air flow 3 by the filter element 10, which comprises activated carbon, ion exchange resin, and/or other active materials. The filtered air flow 3' now passes the sensor unit 20, which is used to check the effectiveness of the filter element 10. The sensor unit 20 is configured to detect the harmful gases (e.g., NO2, NH3, and/or SO2) in the ppm range and also in the ppb range. The filtered air flow 3' enters the fuel cell 2, where the oxygen in the air flow 3' reacts with an energy carrier such as hydrogen 4 to form water 5, thereby generating electrical energy 6.

Figure 2 schematically shows the sensor unit 20. The sensor unit 20 comprises a first sensor 21, a second sensor 22 and a processing unit 23. In the processing unit 23, signals 24, 25 from the sensors 21, 22 are processed into an output signal 26. The output signal 26 can, for example, be transmitted to a further processing device (not shown) via a suitable interface. The output signal 26 can then be used, for example, to obtain the information that a harmful gas concentration determined is too high and that the filter element 10 must be replaced as a result.

Figure 3 schematically shows the structure of the first sensor 21. The structure of the second sensor 22 corresponds to the structure of the first sensor 21, so that only the structure of the first sensor 21 is discussed. The sensor 21 has a first electrode 27 and a second electrode 28, which are arranged on a plate-shaped carrier 29 made of glass. Preferably, the electrodes are made of gold.

The sensor 21 has also a material layer 30 made of polyaniline, which electrically connects the two electrodes 27, 28. The material layer 30 comprises individual layers or films. Polyaniline is an electrically conductive polymer. When the material layer 30 comes into contact with NH3, NO2, and/or SO2, which may be contained in the air flow 3', the electrical conductivity of the material layer changes, so that the resistance between the first electrode 27 and the second electrode 28 changes. The change in resistance can be measured via electrical lines 31.

The material layer 30 of the first sensor 21 differs from the material layer 30 of the second sensor 22 in that the first sensor 21 with the material layer 30 is immersed in an aqueous solution of sulphuric acid for a certain period of time. This immersion in the aqueous solution changes the surface of the material layer 30 of the first sensor 21 compared to the untreated surface of the material layer of the second sensor 22.

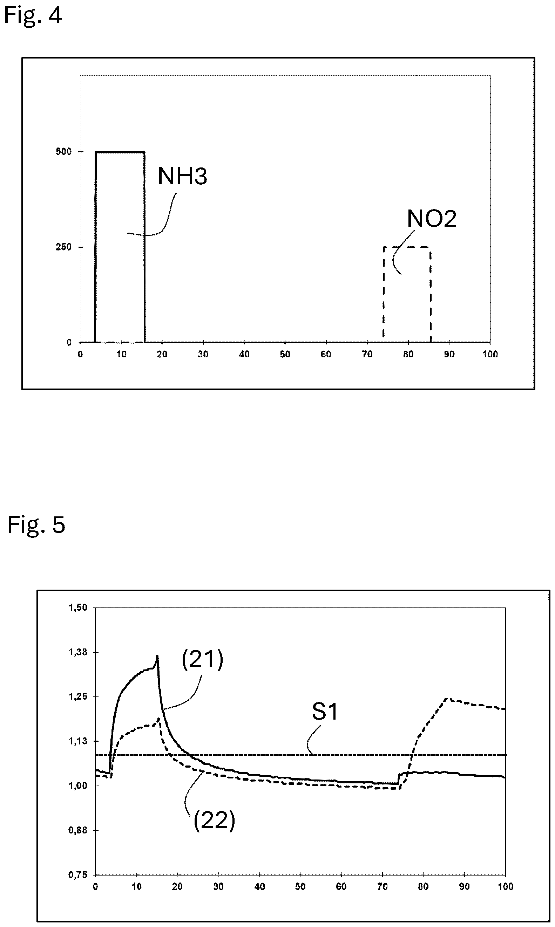

For test purposes, the sensor unit 20 is exposed to the harmful gas NH3 and, with a time delay, to the harmful gas NO2. The diagram in Figure 4 shows the selected concentration in ppb, the exposure duration (10 min) and the time interval (60 min) between the first exposure to NH3 and the second exposure to NO2. The air flow 3' is guided to the two sensors 21, 22 with an NH3 concentration of 500 ppb and, after a break of 60 minutes, with an NO2 concentration of 250 ppb.

The solid line in the diagram in Figure 5 corresponds to the reaction of the first sensor 21. The dashed line corresponds to the sensor reaction of the second sensor 22. The time in minutes is plotted on the x-axis and the relative change in resistance R/RO on the y-axis, where R0 corresponds to the initial resistance of the material layer of the two sensors 21 and 22 before exposure to the harmful gas. It can be seen that the first sensor 21, which was post-treated with H2SO4, shows a stronger reaction when exposed to NH3 than the second sensor 22, which was not post-treated with H2SO4. After exposure to NH3, the sensor signal of the two sensors 21 and 22 returns to essentially the same level.

When exposed to NO2, the sensor 21 post-treated with H2SO4 shows practically no reaction, while the untreated sensor 22 shows a clear reaction (the relative change in resistance R/RO increases to approx. 1.25). After the increase to the value 1.25, however, the relative change in resistance remains at this level and does not return to anywhere near the original value of 1.0. A threshold value S1 for the relative change in resistance R/RO is shown as an example in the diagram in Figure 5. The threshold value S1 here is approx. 1.1. While this threshold value is reached by both sensors when NH3 is applied, only the sensor signal of the untreated sensor 22 is above this threshold value S1 when NO2 is applied.

Based on the comparison with the threshold value S1 and the sensor signal of the two sensors 21, 22, a statement could be made in real operation as to whether the sensor unit has been exposed to NH3 or NO2. If both the first sensor 21 and the second sensor 22 show a sensor response above the threshold value S1, it can be concluded that the harmful gas is NH3. The processing unit can thus generate a corresponding output signal in response to the change in the electrical resistance of the material layer 30 of each of the first sensor 21 and the second sensor 22 being greater than the threshold value S1.

If, on the other hand, only the second sensor 22 shows a reaction above the threshold value S1, it can be concluded that the harmful gas is NO2. The processing unit would now generate a correspondingly different output signal in response to the change in the electrical resistance of the material layer 30 of only the second sensor 22 being greater than the threshold value S1. The above principles can also be applied to detecting SO2 with a third sensor (not shown).

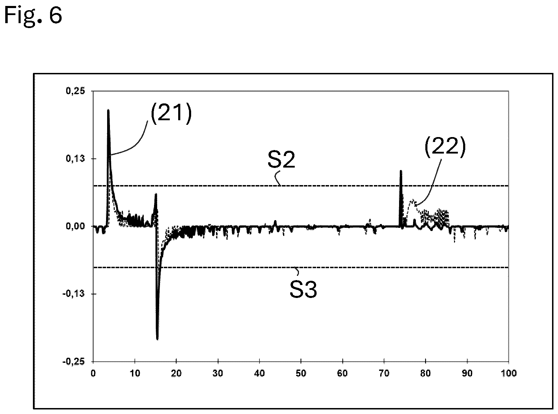

Figure 6 shows the course of the time derivative of the relative resistance change Δ(R/R0)/Δt over time. As in Figure 5, the corresponding sensor response as a result of the exposures shown in Figure 4 is also shown here.

Figure 6 clearly shows that a bipolar pulse can be assigned to both sensors when exposed to NH3. Both the first sensor (post-treated with H2SO4) and the second sensor 22 show a pulse peak at the beginning of the NH3 exposure. When this exposure ends, a negative pulse peak can be recognized, which corresponds approximately to the absolute amount of the initial positive pulse peak.

Exposure to NO2, on the other hand, only results in a unipolar pulse. A negative pulse peak at the end of the NO2 exposure is not recognizable.

In the diagram in Figure 6, a second threshold value S2 is shown as an example for the positive pulse peaks and a third negative threshold value S3 for the negative pulse peaks. The amount of the threshold values can be the same or different. If both sensors 21, 22 now each detect a bipolar pulse, whereby the respective positive pulse peak is above the second threshold value S2 and/or the respective negative pulse peak is below the third threshold value S3, this is an indication of the harmful gas NH3. By checking the corresponding conditions, the processing unit can generate the output signal in response to the time derivative of the change in the electrical resistance of the material layer 30 of each of the first sensor 21 and the second sensor 22 being greater than the threshold value S2 and/or being less than the threshold value S3.

If the sensor signal from the two sensors 21, 22 does not have a negative pulse peak (condition relating to threshold value S3 not fulfilled) after a predefined time window and at the same time the condition relating to the relative resistance change R/RO is met, i.e. the sensor signal from the second sensor 22 is greater than S1 and the sensor signal from the first sensor 21 is less than S1, the processing unit determines an output signal that indicates the presence of NO2. The above principles can also be applied to detecting SO2 with a third sensor (not shown).

Determining the time derivative of the relative change in resistance enables a better and more accurate statement to be made about the harmful gases detected. Due to the usually slowly changing temperature and humidity conditions, mathematical compensation of the sensor signals is not necessary when using the time derivative Δ(R/R0)/Δt. This results in a simple sensor unit with high measurement accuracy. Due to the surface treatment of the material layer of only one sensor with an otherwise identical structure of the material layer of both sensors, the processing unit of the sensor unit can differentiate between NH3, NO2, and/or SO2.

### Reference Signs List

- 1: air filter
- 2: fuel cell
- 3: air flow (3' filtered air flow)
- 4: hydrogen
- 5: water
- 6: energy

- 10: filter element

- 20: sensor unit
- 21: first sensor
- 22: second sensor
- 23: processing unit
- 24: sensor signal
- 25: sensor signal
- 26: output signal
- 27: first electrode
- 28: second electrode
- 29: carrier
- 30: material layer
- 31: line

## Claims

1. A sensor unit (20) for an air filter (1) configured to separate, from an air flow (3), at least one harmful gas, in particular for a fuel cell (2), the sensor unit (20) comprising:
a first sensor (21) and a second sensor (22), each of which comprising two electrodes (27, 28) and a material layer (30) of polyaniline connecting the two electrodes (27, 28), an electrical resistance of the material layer (30) changing when the material layer (30) comes into contact with the harmful gas, and the material layer (30) of the first sensor (21) being different from the material layer (30) of the second sensor (22); and
a processing unit (23) configured to determine a time derivative of a change in the electrical resistance of the material layer (30) of at least the first sensor (21), and to generate an output signal (26) based on the determined time derivative.

2. The sensor unit (20) according to claim 1, **characterized in that** the sensor unit (20) is configured to generate the output signal (26) based on the change in the electrical resistance of the material layer (30) of the first sensor (21) exceeding a threshold value S1.

3. The sensor unit (20) according to claim 1 or 2, **characterized in that** the sensor unit (20) is configured to generate the output signal (26) based on the time derivative of the change in the electrical resistance of the material layer (30) of the first sensor (21) exceeding a threshold value S2.

4. The sensor unit (20) according to one of claims 1 to 3, **characterized in that** the sensor unit (20) is configured to generate the output signal (26) based on the time derivative of the change in the electrical resistance of the material layer (30) of the first sensor (21) falling below a negative threshold value S3.

5. A method of manufacturing the sensor unit (20) according to one of claims 1 to 4, the method comprising subjecting the material layer (30) of the first sensor (21) to a surface treatment with an acid.

6. The method according to claim 5, **characterized in that** the acid comprises one among sulphuric acid (H2SO4), hydrochloric acid (HCl), nitric acid (HNO3), phosphoric acid (H3PO4), and sulphonic acid.

7. The method according to claim 5 or 6, **characterized in that** the surface treatment comprises immersing the material layer (30) of the first sensor (21) in a 1-molar to 10-molar aqueous solution of the acid.

8. The method according to claim 7, **characterized in that** the material layer (30) is immersed in the acid for 5 to 30 seconds.

9. The method according to one of claims 5 to 9, further comprising building up the material layer (30) by applying a dynamic potential to aniline in an aqueous solution of the acid so that the aniline is polymerised.

10. The method according to claim 9, further comprising functionalizing the material layer (30) by mixing one among metals, metal alloys, and metal oxides into the aqueous solution.

11. The method according to claim 9 or 10, further comprising performing chemical metal deposition on the material layer (30) by immersing the material layer (30) in a dispersion comprising a solvent and metal nanoparticles.
